# EUROPEAN PATENT APPLICATION

(11) **EP 2 100 640 A1**
(43) Date of publication of application: **16.09.2009**
(21) Application number: 08169931.6
(22) Date of filing: 11.02.2004
(51) Int. Cl.: A61N 5/067, A61N 5/06

(54) **Near infrared microbial elimination laser system**

(30) Priority: 26.08.2003 US 649910
(62) Divisional of application: 04710257.9
(71) Applicant: Bornstein, Eric, Natick, MA 01760 (US)
(72) Inventor: Bornstein, Eric, Natick, MA 01760 (US)
(74) Representative: Rickard, David John

(57) **Abstract**

Dual wavelength laser energy in the low infrared electromagnetic spectrum destroys bacteria via photo-damage optical interactions through direct selective absorption of the optical energy by intracellular bacterial chromophores. The dual wavelength laser system (22) includes an optical assembly including two distinct diode laser ranges (870 nm diode array and 930 nm diode array) that can be emitted to achieve maximal bacterial elimination.

## Description

### BACKGROUND OF THE INVENTION

### Field of the Invention

The present invention relates to off-site and on-site destruction of bacteria, and, more particularly, to the in-vivo destruction of bacteria by laser energy in medical, dental and veterinary surgical sites, as well as other sites in biological or related systems.

### The Prior Art

Traditionally solid state diode lasers in the low infrared spectrum (600 nm to 1000 nm) have been used for variety of purposes in medicine, dentistry, and veterinary science because of their preferential absorption curve for melanin and hemoglobin in biological systems. They rarely, if at all, have been used for sterilization outside of biological systems.

Because of poor absorption of low infrared diode optical energy in water, low infrared penetration in biological tissue is far greater than that of higher infrared wavelengths.

Specifically, diode laser energy can penetrate biological tissue to about 4 cm. In contrast, Er:YAG and CO₂ lasers, which have higher water absorption curves, penetrate biological tissue only to about 15 µm and 75 µm, respectively (10,000 µm = 1 cm).

Therefore, with near infrared diode lasers, heat deposition is much deeper in biological tissue, and more therapeutic and beneficial in fighting bacterial infections. However, to prevent unwanted thermal injury to a biological site being irradiated, the radiance (joules/cm²) and/or the exposure time of diode lasers must be kept to a minimum.

For the accomplishment of bacterial cell death with near infrared diode lasers in biological systems, the prior art is characterized by a very narrow therapeutic window. Normal human temperature is 37°C, which corresponds to rapid bacterial growth in most bacterial infections. When radiant energy is applied to a biological system with a near infrared diode laser, the temperature of the irradiated area starts to rise immediately, with each 10°C rise carrying an injurious biological interaction. At 45°C there is tissue hyperthermia, at 50°C there is a reduction in enzyme activity and cell immobility, at 60°C there is denaturation of proteins and collagen with beginning coagulation, at 80°C there is a permeabilization of cell membranes, and at 100°C there is vaporization of water and biological matter. In the event of any significant duration of a temperature above 80°C, (five to ten seconds in a local area), irreversible harm to the biological system will result.

To kill bacteria by photothermolysis (heat induced death) in the prior art, a significant temperature increase must occur for a given amount of time in the bacteria-containing site. With traditional near infrared diode optical energy, it is desired to destroy bacteria thermally, without causing irreversible heat induced damage to the biological site being treated.

### SUMMARY OF THE INVENTION

The near infrared microbial elimination laser (NIMEL) system, process and product of the present invention utilize a dual wavelength, near-infrared, solid state diode laser combination, preferably but not necessarily, in a single housing with a unified control. They involve emission of radiation in two narrow ranges approximating 870 nm and 930 nm. They are most effective when the radiation is substantially at 870 nm and 930 nm. It has been found that these two wavelengths interactively are capable of selectively destroying E. coli with non-ionizing optical energy and minimal heat deposition. The laser combination of the present invention, which emits these wavelengths simultaneously or alternately, and continuously or intermittently, preferably incorporates at least one ultrashort pulse laser oscillator, composed of titanium-doped sapphire.

The system, process and product of the present invention are widely applicable in medical and dental surgery, and in water purification, agriculture, and in emergency and military scenarios.

### BRIEF DESCRIPTION OF THE DRAWINGS

For a fuller understanding of the nature and object of the present invention, reference is made to the accompanying drawings, wherein:
Fig. 1a illustrates the design, partially diagrammatically, of dental instrumentation embodying the present invention;
Fig. 1b is a block diagram of the laser oscillators and control system of the instrumentation of Fig. 1a;
Fig. 2a shows details of a laser energy delivery head for the instrumentation of Fig. 1a;
Fig. 2b shows details of an alternative laser energy delivery head for the instrumentation of Fig. 1a;
Fig. 3a shows wavelength division multiplexing details of the laser system of Fig. 1;
Fig. 3b shows further wavelength division multiplexing details of the laser system of Fig. 1;
Fig. 4a is a block diagram of a surgical process embodying the present invention;
Fig. 4b is a block diagram of another surgical process embodying the present invention;
Fig. 5 depicts selected chromophore absorption leading to bacterial cell death pursuant to the present invention;
Fig. 6 depicts the application of the present invention to a periodontal pocket;
Fig. 7a is an illustration of a laser augmented periodontal scaling instrument (LAPSI) embodying the present invention;
Fig. 7b is a broken-away illustration showing details of the head of the instrument of Fig. 7a;
Fig. 7c is a broken-away illustration showing details of one embodiment of a blade of the instrument of Fig. 7a;
Fig. 7d is a broken-away illustration showing details of another embodiment of a blade of the instrument of Fig. 7a;
Fig. 8 illustrates an application of the present invention to a root canal procedure;
Fig. 9 illustrates an application of the present invention to a gangrenous condition of a finger, toe or recalcitrant diabetic ulcer;
Fig. 10 illustrates an application of the present invention to an ear infection;
Fig. 11 illustrates an application of the present invention to a bandage for destroying bacteria on the human body; and
Fig. 12 illustrates an application of the present invention to a wand for destroying bacteria on the human body.

### DETAILED DESCRIPTION OF THE INVENTION

The present invention is based upon a combination of insights that have been introduced above and are derived in part from empirical facts, which include the following.

Most infectious bacteria, when heated, continue growing until their temperature reaches approximately 50°C, whereupon their growth curve slows.

At approximately 60°C, bacterial growth comes to an end, except in cases of the hardiest bacterial thermophiles.

The range of approximately 60°C to approximately 80°C is generally accepted as the time dependent exposure necessary for bacterial death.

Hence, in the prior art, there has been a very narrow window of therapeutic opportunity to destroy the bacteria with heat from a traditional near infrared diode laser (60°C to 80°C) without causing irreversible heat induced damage (more than five seconds) to the biological site being treated.

The dual wavelength, solid state, near-infrared diode laser system of the present invention is specifically designed for bacterial destruction with minimal heat deposition in the site being irradiated. It has been found that the wavelength combination of the present invention is capable of destroying bacterial cells such as E. coli as a result of the interaction of a toxic singlet oxygen reaction that is generated by the absorption of laser energy selectively in intracellular bacterial chromophores. These chromophores happen to be specific to wavelengths that approximate 870 nm and 930 nm in the near infrared spectrum.

Without the significant heat deposition normally associated in the prior art with continuous wave or pulsed near infrared diode lasers, bacteria can be selectively destroyed while minimizing unwanted hyperthermia of the irradiated tissues and the surrounding region. The system, process and product of the present invention are based on a study of facts derived from research conducted with the technology of so-called optical cell trapping and optical tweezers.

Optical tweezers are near infrared based optical traps (created for cell biology), which simply use infrared laser beams of very low power to hold and study single cells of various prokaryotic and eukaryotic species while keeping them alive and functional under a microscope. When this procedure is effected with near infrared laser energy, intense heat deposition generally occurs. To accomplish the goal of "holding" a single cell in place without killing it by thermolysis, the laser energy must be reduced to under 100 milliwatts of energy. Thereby, the bacteria may be kept alive for a five minute period or longer.

In an elegant study using a tunable Ti:Sapphire laser, Neuman (Biophysical Journal, Vol. 77, November 1999) found that, even with this very low laser output to rule out direct heating (thermolysis) as the source of bacterial death, there are two distinct and only two distinct wavelengths in the near infrared spectrum, which cannot be used successfully for optical traps because of their lethal affect on E-coli bacteria. These wavelengths are 870 nm and 930 nm. Neuman found that the two wavelengths, 870 nm and 930 nm (in contrast to all others in the near infrared spectrum), are not transparent to the bacteria being studied.

He postulated that the two wavelengths probably interact with a linear one photon process mediated through absorption of one or more specific intracellular bacterial chromophores or pigments. This one photon process of photodamage (not thermal damage) to the bacteria, he further concluded, implies a critical role for a short acting singlet oxygen species, or a reactive oxygen species as the culprit in the cellular damage pathway.

Accordingly, the system, process and product of the present invention are characterized by the following general considerations.

The present invention provides a dual wavelength diode laser combination for bacterial destruction with minimal heat deposition in human medicine and dentistry, veterinary medicine, water purification, agriculture, and military scenarios.

If used in any medical, biological, military or industrial system, this combination of diode oscillators can be used singly or multiplexed together to effect maximal bacterial death rates in the site being irradiated.

In various embodiments, the energies from both diode laser oscillators preferably are conducted, either singly or multiplexed, along a common optical pathway to effect maximal bacterial death rates in the site being irradiated.

In certain alternative embodiments, the energies from both diode laser oscillators are delivered separately, simultaneously or alternately through multiple optical pathways.

In accordance with the present invention, it is critical that the laser wavelengths selected as approximating 870 nm and 930 nm, respectively, lie predominantly within the wavelength ranges of (1) 865 nm to 875 nm and (2) 925 nm to 935 nm.

Instead of avoiding the 870 nm and 930 nm wavelengths as suggested in the prior art by optical tweezer procedures, the laser system and process of the present invention selectively combines them. With less heat deposition in the site being irradiated, a much enlarged therapeutic window of opportunity is available to the laser operator. In essence, the combined wavelengths of the present invention use less energy than do prior art procedures to effect bacterial destruction, i.e. the optical energy used in the present invention is less than the thermal energy used in the prior art.

The medical, dental or veterinary applications of the dual wavelength combination of the present invention include, but are not limited to, coagulation, tissue vaporization, tissue cutting, selected photodynamic therapy, and interstitial thermal-therapy, and selected bacterial destruction.

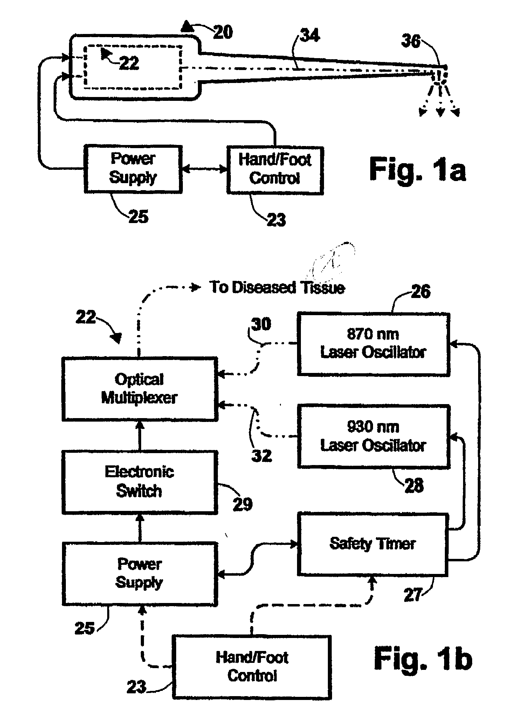

### Figs. 1a to 3b: The Dual Wavelength System

A laser system for destroying bacteria in a bacterial dental site is shown in Figs. 1a-3b as comprising a housing 20 and a laser system 22. Within the housing is a laser oscillator sub-system 26, 28 for causing the selective emission of radiation 30 in a first wavelength range of 865 nm to 875 nm, and the selective emission of radiation 32 in a second wavelength range of 925 nm to 935 nm. It is to be understood that, in alternative embodiments, a group of laser oscillators are employed in tandem in accordance with the present invention. The radiation is propagated through an optical channel 34 to a head 36 for enabling delivery of the radiation through the optical channel to a bacterial site.

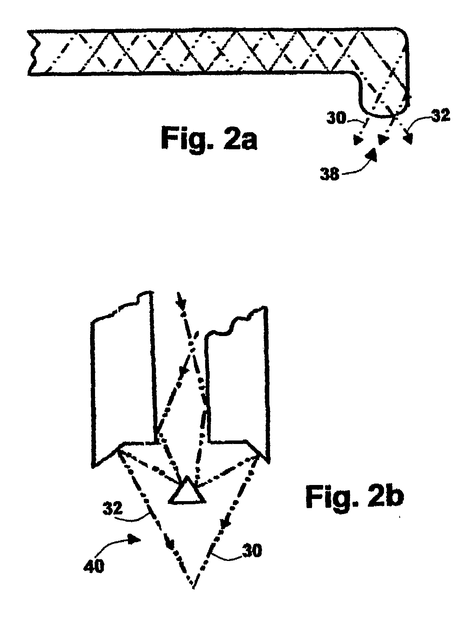

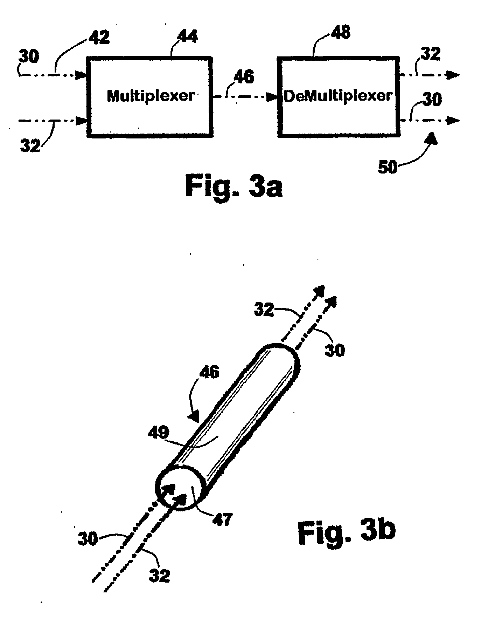

In various delivery systems, the delivery is disperse as shown at 38 in Fig. 2a or focused as shown at 40 in Fig. 2b. In another version, parts of which are shown in Figs. 3a and 3b, the laser oscillators are deployed outside of housing 20 as at 42, are multiplexed as at 44, transmitted via a coaxial cable as at 46, de-multiplexed as at 48, and delivered via a housing as at 50. Coaxial cable 46 is shown in physical form in Fig. 3b as including a glass fiber 47 and a cladding 49.

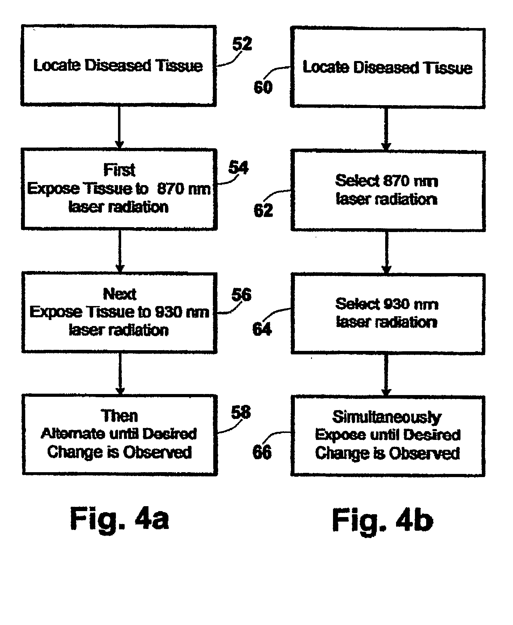

### Figs.4a, 4b, 5 and 6: The Process

One process of the present invention is shown in Fig. 4a as including the steps of locating diseased tissue as at 52, exposing the tissue to 870 nm laser radiation as at 54, exposing the tissue to 930 nm radiation as at 56, and alternating the two exposures as at 58 until desired change is observed or cultured.

Another process of the present invention is shown in Fig. 4b as including the steps of locating diseased tissue as at 60, simultaneously exposing the diseased tissue to 870 nm laser radiation at 62 and 930 nm laser radiation at 64, and maintaining the exposure until desired change is observed or cultured.

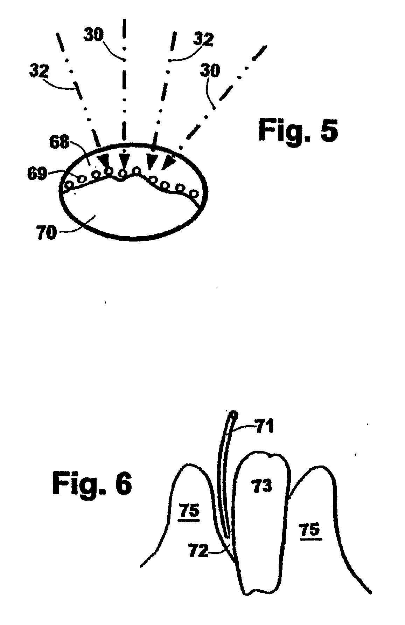

Generally, as shown in Fig. 5, the two wavelengths activate a chromophore 68, activate the chromophore at the diseased site, and then cooperate with the chromophore at 70 to destroy the bacteria.

This process is capable of wide application as in Fig. 6, wherein, the two laser wavelengths of the present invention are transmitted through a 600 µm fiber optic channel 71 in the therapeutic treatment of a deleterious ecological niche known as a periodontal pocket 72, between tooth 73 and gum 75 to achieve bacterial elimination and limit the use of antibiotics.

### EXAMPLE I

The prior art literature (Neuman, Biophysical Journal, Vol. 77, November 1999, *infra*) reports that 870 nm and 930 nm radiation from a tunable Ti:Sapphire laser during confocal microscopy has produced a 7-fold mortality in E. coli. A careful study of this information by the inventor hereof has lead to the following conclusions. At face value, it is power density (brilliance) that, aside from the 870 nm and 930 nm wavelengths, is the most important parameter to cause the above described toxic singlet oxygen reaction. This can be calculated using the formula: Power density (W/cm²) = total power (W) X spot size (cm²). Using this relationship, it is calculated that, with at least 100 mW and an adjustment of spot size, necessary bactericidal density can be reached. It is believed that the toxic singlet oxygen reaction takes place in accordance with a power density curve. It is adjustable by increasing power (always below tissue coagulation potential), by increasing spot size, or by scanning the tissue with a set spot of high intensity and minimal size. The mortality ratio is directly proportional to power density increase. It is not necessary to kill all bacteria. It is necessary only to kill sufficient bacterial to enable the body's immune system to the rest.

### EXAMPLE II

The unique bactericidal capabilities of 870 nm and 930 nm radiation may be demonstrated by the following equation, which considers the wave nature of light, the energy per photon based on wavelength, and what that energy does to cells: E = hf, where E = energy, h = Plank's constant, and f = speed of light/wavelength. E = hf really describes a photon's momentum. In other words, a photon's momentum is directly related to energy. This means, the shorter the wavelength, the greater the momentum (energy) of the photon. Consider the following.

### Ultraviolet Wavelengths

1) ArF laser at 193 nm generates UV-C at 6.4 electron volts/photon (EV/photon)
2) XeCl laser at 308 nm generates UV-A at 4.0 EV/photon

### Visible Wavelengths

1) Ar laser at 514 nm generates 2.4 EV/photon
2) He-Ne Laser at 633 nm generates 2.0 EV/photon

### Infrared Wavelengths

1) Diode laser at 800 nm generates 1.6 EV/photon
2) Er:Yag Laser at 2940 nm generates 0.4 EV/phot on
3) CO₂ laser at 10600 nm generates 0.1 EV/photon

Hence, the shorter (UV) wavelengths, because of their frequency, are more energetic than the longer wavelengths. And less energy per photon is generated as the wavelength rises into the visible and then the infrared regions of the electro-magnetic spectrum.

### EXAMPLE III

It is well known that: (1) ultraviolet light and ultraviolet lasers are more highly energized than visible or infrared, and that they "in and of themselves" are mutagenic in nature; (2) ultraviolet (non-ionizing) radiation of greater than six EV/photon (e.g., UV ArF) can excite electrons in a biomolecule (e.g., DNA) into an ionization state; (3) less than six EV/photon (UV-A, UV-B, visible, and infrared) can only excite biomolecule electrons into higher electronic or vibrational states, but not ionization states, because the photons carry substantially less energy; (4) UV-B and UV-A can cause substantial cross-link damage without ionization, again because of the extra electron volts that they carry at this non-ionizing UV wavelength.

It is exactly these higher energy ionization states caused by certain higher energy UV photons (UV-C) upon absorption by biomolecules, that can cause pyrimidine dimers in the DNA.

The 870 nm and 930 nm energy, independently of energy density, only produce photons that carry 1.4 - 1.6 EV/photon, i.e., less than the energy that will cause DNA damage, but still lethal at 100 mW power densities to E. coli. At such a power density, Neuman found the toxic singlet oxygen reaction (from selective chromophore absorption) that kills E. coli. This most likely happens by selectively exciting biomolecule (the chromophore) electrons into a higher vibrational state, and liberating the singlet oxygen.

The eukaryotic CHO (Chinese Hela Ovary) cell also studied by Neuman and affected by these wavelengths, in general, are far more fragile cells than human skin, muscle, and connective tissue. It is yet to be seen what selective power densities will do to these cells in a negative manner, but, as the above considerations demonstrate on an empirical level, over the years, many energies approaching 870 nm and 930 nm, at energy densities that normally are high enough to burn tissue, have been tested and considered safe to human tissue. Human tissue generally "bounces back" from years of repetitive UV sun burns. In comparison, it is concluded that 870 nm and 930 nm infrared energy is toxic to certain microbes and probably just bothersome to the human tissues.

### EXAMPLE IV

The bactericidal effects of 870 nm and 930 nm energy on E. coli are known on the basis of empirical tests. Although, as far as is known, no such tests with these wavelengths have been performed on other bacteria, it is probable that bacteria other than E. coli will be affected similarly. This probability is based upon the following logic. Antibiotics are developed to address specific necessary bacterial systems that differ from specific necessary human systems. Examples of this principle follow:
Penicillins: All address an enzyme that helps build a peptidoglycan cell wall in a range of bacteria. This is a ubiquitous event that is inconsequential to humans and animals, because they do not have cell walls.
Erythromycins: All inhibit protein synthesis in a range of bacteria by disturbing their bacterial ribosome subunits in most bacteria. Bacterial ribosome is different from the human and animal ribosomes, so such disturbance does no harm to humans and animals.
Tetracyclines: All inhibit a different aspect of bacterial protein synthesis.
Ciprofloxin: This inhibits a bacterial enzyme called DNA gyrase, which allows the bacterial DNA to unfold for bacterial replication and protein synthesis. This is an enzyme that is different from any human enzyme, so it has no corresponding effect on humans.

There are more similarities in bacteria than there are differences. If penicillin or erythromycin worked only on three or four bacterial species, and were not "broad spectrum" in nature, they would be far less useful. However, they generally work across the board, because so much is similar in the biochemistry and morphology of a vast majority of bacteria. The conclusion is that there is wide applicability of bacterial destruction by 870 nm and 930 nm infrared radiation. This conclusion is based on the logic that the chromophore these wavelengths address in E. coli, which causes the toxic singlet oxygen reaction, is present in many more species than only E. coli.

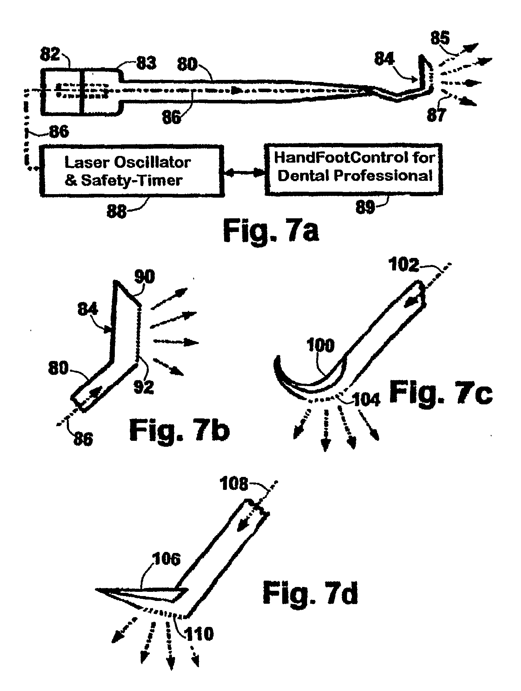

### Figs. 7a to 7d: Laser Augmented Dental Scaling

Dental instruments are designed for the purpose of removing calculus and plaque, root planing, and removing diseased soft tissues from periodontal pockets and the like. The illustrated radiation and scaling instruments of the present invention generally comprise (1) a shank which is to be hand held and manipulated by a dental professional during an operation, (2) at least one working end which presents, in contiguity, a laser optical head and a mechanical cutting head that simultaneously address a surgical site, and (3) a fiber optic laser bundle that extends from an optical input at one end of the shank, at which a laser is fitted, to an optical output at the other end of the shank, at which laser energy is delivered. The arrangement is such that, during an operation, the dental professional can subject the surgical site simultaneously or alternately to (1) mechanical cutting, scraping and grinding, and (2) laser trimming and cauterization.

Generally, the shank is composed of stainless steel, high carbon steel, and/or autoclaveable high strength plastic (for implants). The laser connects through an interchangeable fitting to a conventional the fiber optic bundle in or at the shank. The fiber optic bundle, when located in the shank, allows optical energy to exit in contiguity with the head through a heat and scratch resistant quartz window, where, upon exit, it bathes the surgical site, e.g., a periodontal pocket and tissues, with diode laser energy.

Fig. 7a illustrates a curette comprising, in accordance with the present invention: a hollow shank 80 having a rearward interchangeable fitting 82, and a forward contact head 84. Within shank 80 extends a fiber optic bundle 86. As shown, laser energy 85, 87 is delivered from safety-timed laser oscillators 88 through an interchangeable fitting 82 and laser bundle 86 to contact head 84 under a hand/foot control 89. As shown in Fig. 7b, in contiguity at contact head 84 are a blade 90 and an exit window 92.

As shown in Figs. 7c and 7d, respectively, one embodiment of the blade is curved as at 100, and another embodiment of the blade is linear as at 106. In the embodiment of Fig. 7c, fiber optic bundle 102 and window 104 closely underlie the cutting edge of the blade. In the embodiment of Fig. 7d, fiber optic bundle 108 and window 110 closely underlie the cutting edge of the blade. Each of the scalers of Figs. 7c and 7d has a mating fitting 83 that is attachable to mating fitting 82 for optional and interchangeable communication with the two laser oscillators.

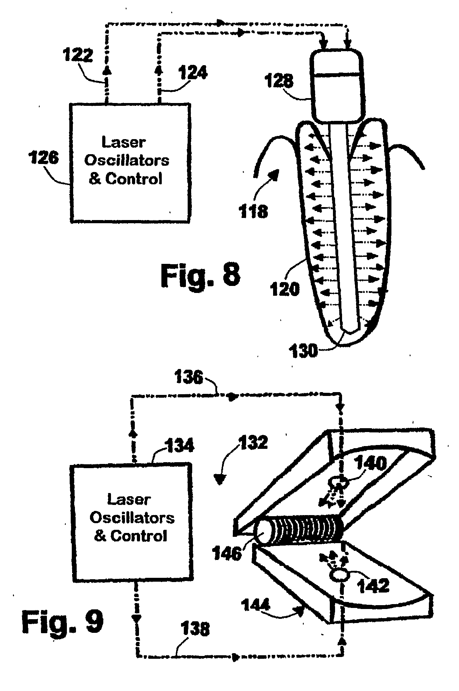

### Fig. 8: Laser Augmented Root Canal Therapy

Fig. 8 illustrates a system 118 which is designed for use in the therapeutic treatment of bacteria in the root canal of a tooth. The objective is to provide targeted energy for infected root canal space within a tooth to achieve bacterial elimination within the dentinal tubules.

As shown, dual wavelength energy 122, 124 of the present invention is generated at 126, fed through an optical coupling 128, and dispersed through a laser augmented root canal interstitial thermal therapy tip 130, which is frosted with sapphire or silica granules. As a result, bacterial elimination in the root canal is achieved and the need for conventional antibiotics is ameliorated or obviated.

### Fig. 9: Treatment of Gangrenous Fingers and Toes

Fig. 9 shows a system 132 embodying the present invention for use as an adjunct to treat infected and gangrenous fingers and toes in diabetic patients. In the preferred embodiment for this approach, the dual wave length energy is generated at 134, is fed through optical channels 136 and 138, and is dispersed through opposed dual apertures 140 and 142 in a clip 144. The clip 144, which is spring loaded at 146, is clamped on the diseased digit (finger or toe) of a patient and bathes an infected area of a finger or toe with the dual wave length energy for a defined period at a defined power to effect bacterial elimination without detrimental heat deposition.

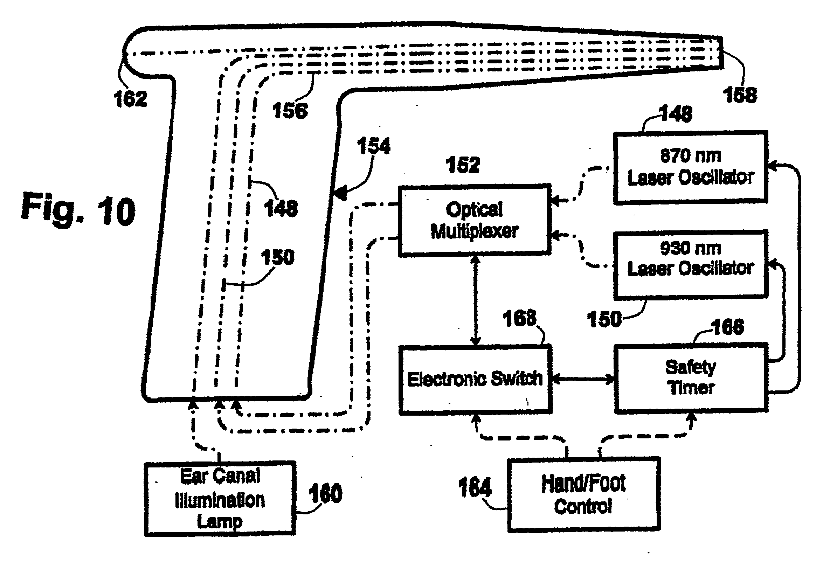

### Fig. 10: Laser Augmented Otoscope

Fig. 10 shows the therapeutic use of 870 nm energy 148 and 930 nm energy 150 in accordance with the present invention as an adjunct for curing otitis media (ear infections). As shown, the dual wavelength energy is channeled by an optical multiplexer 152 through an otoscope 154 having an optical channel 156 for conduction of the energy to an optical head 158 that may be inserted into the ear canal. This allows the practitioner, under direct illumination from a lamp 160 and visualization at an eye piece 162, to irradiate the inner ear drum and canal with dual laser energy to effect bacterial elimination in the ear canal and inner ear without thermal tissue destruction. A hand/foot control manages the operation via a safety timer 166 and an electronic switch 168.

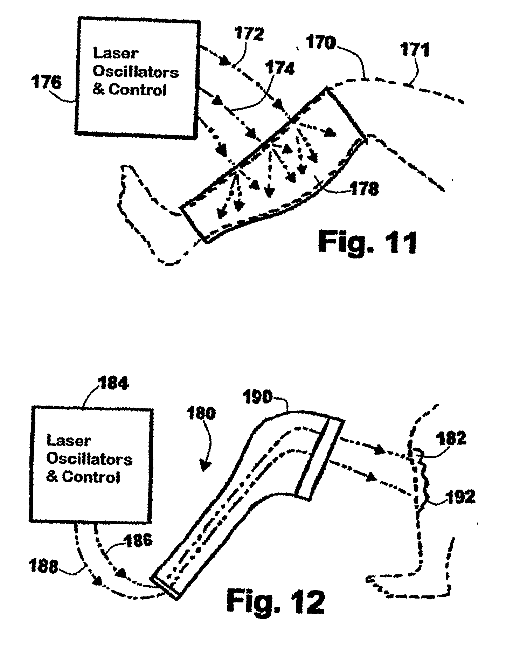

### Fig. 11: Laser Augmented Therapeutic Wrap

Fig. 11 shows a system 170 embodying the present invention for use as an adjunct for the treatment of a limb 171 that is infected with cellulites, necrotizing fasciitis, or other dermatological disease. As shown, dual wavelength energy 172,174 of the present invention is generated at 176 and transmitted to a fiber optic illuminating fabric 178 for distributed irradiation of the limb. This fabric incorporates erratically clad optical fibers typically 200 to 400 µm in diameter, which deliver the dual wave length energy to the diseased region of the limb for the eradication of bacteria.

### Fig. 12: Therapeutic Wand

Fig. 12 shows a system 180 for applying the dual wave length energy of the present invention for bacterial elimination of an infected wound or surgical site. The dual wavelength energy is generated at 184 for transmission at 186 and 188 to a hand-held wand 190. Under manual controls in the handle of the wand, the 870 nm and 930 nm wave lengths are applied simultaneously or alternately to a wound or infection as at 192 to accomplish bacterial destruction optically. This instrument is adapted for use in a hospital setting or in conjunction with a battery powered field pack for military triage.

### OPERATION

In operation, each of the illustrated embodiments is capable of generating continuous wave or pulsed laser energy independently or at the same time depending on the parameters set by the operator. To this laser is connected to a suitable fiber optic delivery system. This system generates from 100 mW to 20 W of laser output from each wavelength independently or a total of 200 mW up to 40 W together depending on the parameters set by the operator. By using the bacteria's own chromophores, the system produces maximum lethal effects on the bacteria with minimal heat deposition.

The purposes of such radiant exposure, in various embodiments, are ablation of tissue, vaporization of tissue, coagulation of a surgical area, photochemical interactions, and bacterial death by thermolysis of bacterial cells. Infrared radiation is known as "heat radiation" because it directly generates heat for bacterial destruction, i.e., thermolysis. The present invention accomplishes bacterial destruction by optical energy, i.e., photolysis rather than thermolysis.

Since certain changes may be made in the present disclosure without departing from the scope of the present invention, it is intended that all matter described in the foregoing specification and shown in the accompanying drawings be interpreted as illustrative and not in a limiting sense.

## Claims

1. A laser system for destroying bacteria in a bacterial locale, said system comprising:
(a) a housing and a control;
(b) a laser oscillator sub-system within said housing for causing the selective emission under said control of first radiation in a first wavelength range of 865 nm to 875 nm, and the selective emission under said control of second radiation at a second wavelength range of 925 nm to 935 nm;
(c) an optical channel for transmission of said first radiation and said second radiation from said laser oscillator sub-system; and
(d) a head for enabling delivery of said first radiation and said second radiation from said laser oscillator sub-system through said optical channel to the site of said bacterial locale;
(e) said first radiation and said second radiation being adapted to activate a chromophore from said bacterial locale and being adapted to cooperate with said chromophore to destroy bacteria in said bacterial locale.

2. The laser system of claim 1 wherein said transmission is simultaneous.

3. The laser system of claim 1 wherein said transmission is alternate.

4. The laser system of claim 1 wherein said transmission is multiplexed.

5. The laser system of claim 1 wherein said head includes an optical egress for said first radiation and said second radiation, and a scaling instrument.

6. The laser system of claim 1 wherein said head includes an optical egress having a frosted tip.

7. The laser system of claim 1 wherein said head includes an optical egress and an otoscope.

8. The laser system of claim 1 wherein said head includes a digit clip and an optical egress therefrom.

9. The laser system of claim 1 wherein said head includes a stocking having an optical ingress from said laser oscillator and an optical egress to the inner surface of said stocking.

10. The laser system of claim 1 wherein said head includes a handle and an optical egress extending therefrom.

11. A laser system for destroying bacteria in a bacterial locale, said system comprising:
(a) a housing and a control;
(b) a laser oscillator sub-system within said housing for causing the selective emission under said control of first radiation narrowly at a first wavelength of substantially 870 nm and the selective emission under said control of second radiation at a second wavelength of substantially 930 nm;
(c) a head for delivering said first radiation and said second radiation from said laser oscillator sub-system to the site of said bacterial locale; and
(d) said first radiation and said second radiation being adapted to activate a chromophore from said bacterial locale and being adapted to cooperate with said chromophore to destroy bacteria in said bacterial locale.

12. The laser system of claim 11 wherein said transmission is simultaneous.

13. The laser system of claim 11 wherein said transmission is alternate.

14. The laser system of claim 11 wherein said transmission is multiplexed.

15. The laser system of claim 11 wherein said head includes an optical egress for said first radiation and said second radiation, and a scaling instrument.

16. The laser system of claim 11 wherein said head includes an optical egress having a frosted tip for insertion into a root canal.

17. The laser system of claim 11 wherein said head includes an optical egress and an otoscope.

18. The laser system of claim 11 wherein said head includes a digit clip and an optical egress therefrom.

19. The laser system of claim 11 wherein said head includes a stocking having an optical ingress from said laser oscillator and an optical egress to the inner surface of said stocking.

20. The laser system of claim 11 wherein said head includes a handle and an optical egress extending therefrom.

21. A process for destroying bacteria in a bacterial locale, said process comprising:
(a) energizing a laser to cause the selective emission of first radiation in a first wavelength range of 865 nm to 875 nm and the selective emission of second radiation at a second wavelength range of 925 nm to 935 nm;
(b) establishing a path for the transmission of said first radiation and said second radiation from said laser oscillator sub-system; and
(c) enabling delivery of said first radiation and said second radiation from said laser oscillator sub-system through said optical channel to the site of said bacterial locale;
(d) said first radiation and said second radiation activating a chromophore from said bacterial locale and cooperating with said chromophore to destroy bacteria in said bacterial locale.

22. A process for destroying bacteria in a bacterial locale, said process comprising:
(a) energizing a laser to cause the selective emission of first radiation in the selected wavelength of 870nm and the selective emission of second radiation in the selective wavelength range of 930nm;
(b) establishing a path for the transmission of said first radiation and said second radiation from said laser oscillator sub-system; and
(c) enabling delivery of said first radiation and said second radiation from said laser oscillator sub-system through said optical channel to the site of said bacterial locale;
(d) said first radiation and said second radiation activating a chromophore from said bacterial locale and cooperating with said chromophore to cause a reaction with bacteria in said bacterial locale.

23. The process of claim 22 wherein said bacteria is E. coli.

24. The process of claim 22 wherein said reaction is a toxic singlet oxygen reaction.

25. A laser process comprising destroying bacteria in an infected locale by a reaction resulting from application to said infected locale of laser radiation, which is primarily of two wavelength ranges that are generated by a laser system:
(a) said bacteria including E. coli;
(b) said system comprising:
(1) a housing and a control;
(2) a laser oscillator sub-system within said housing for causing the selective emission under said control of first radiation that is primarily in a first wavelength range of 865 nm to 875 nm, and the selective emission under said control of second radiation at a second wavelength range that is primarily in a wavelength range of 925 nm to 935 nm;
(3) an optical channel for transmission of said first radiation and said second radiation from said laser oscillator sub-system; and
(4) a head for enabling delivery of said first radiation and said second radiation from said laser oscillator sub-system through said optical channel to the site of said bacterial locale;
(5) said first radiation and said second radiation activating a chromophore from said bacterial locale and cooperating with said chromophore to destroy said bacteria in said bacterial locale.

26. A laser process comprising destroying bacteria in an infected locale by a reaction resulting from application to said infected locale of laser radiation, which is primarily of two wavelength ranges that are generated by a laser system, said system comprising:
(a) a housing and a control;
(b) a laser oscillator sub-system within said housing for causing the selective emission under said control of first radiation that is primarily in a first wavelength range of 865 nm to 875 nm, and the selective emission under said control of second radiation at a second wavelength range that is primarily in a wavelength range of 925 nm to 935 nm;
(c) an optical channel for transmission of said first radiation and said second radiation from said laser oscillator sub-system; and
(d) a head for enabling delivery of said first radiation and said second radiation from said laser oscillator sub-system through said optical channel to the site of said bacterial locale;
(e) said first radiation and said second radiation activating a chromophore from said bacterial locale and cooperating with said chromophore to destroy said bacteria in said bacterial locale;
(f) said reaction being a toxic singlet oxygen reaction.

27. A dental process comprising scaling an infected locale and destroying bacteria in said infected locale by a reaction resulting from application to said infected locale of laser radiation, which is primarily of two wavelength ranges that are generated by a laser system, said system comprising:
(a) a housing and a control, said system comprising a head that includes a dental scaler and an optical egress in close proximity;
(b) a laser oscillator sub-system within said housing for causing the selective emission under said control of first radiation that is primarily in a first wavelength range of 865 nm to 875 nm, and the selective emission under said control of second radiation at a second wavelength range that is primarily in a wavelength range of 925 nm to 935 nm;
(c) an optical channel for transmission of said first radiation and said second radiation from said laser oscillator sub-system;
(d) said head enabling delivery of said first radiation and said second radiation from said laser oscillator sub-system through said optical channel to the site of said bacterial locale;
(e) said first radiation and said second radiation activating a chromophore from said bacterial locale and cooperating with said chromophore to destroy said bacteria in said bacterial locale; and
(f) said reaction being a toxic singlet oxygen reaction.

28. A dental process comprising:
(a) inserting a mechanical probe into an infected root canal to expose said root canal;
(b) removing said mechanical probe from said infected root canal;
(c) inserting an optical probe into said infected root canal to cause a reaction in bacteria in said infected root canal by transmission of laser radiation from said optical probe to bacteria in said infected root canal;
(d) said laser radiation consisting essentially of one or both of a first radiation and a second radiation, said first radiation being primarily in a first wavelength range of 865 nm to 875 nm, and said second radiation being primarily in a second wavelength range of 925 nm to 935 nm;
(e) said first radiation and/or said second radiation activating a chromophore in said bacterial locale and cooperating with said chromophore to destroy said bacteria;
(f) said reaction being a toxic singlet oxygen reaction;
(g) removing said optical probe from said root canal; and
(h) filling said root canal with a dental prosthesis.

29. The process of claim 28 wherein said bacteria is E. coli.

30. A therapeutic process comprising:
(a) inserting a diseased digital member into a clip having a pair of opposed elements;
(b) said opposed elements having optical egresses in communication with opposed sections of said digital member;
(c) causing a reaction in bacteria in said diseased digital member by transmission of laser radiation from said optical egresses to said bacteria;
(d) said laser radiation consisting essentially of one or both of a first radiation and a second radiation, said first radiation being primarily in a first wavelength range of 865 nm to 875 nm, and said second radiation being primarily in a second wavelength range of 925 nm to 935 nm;
(e) said first radiation and/or said second radiation activating a chromophore in said bacteria and cooperating with said chromophore to destroy said bacteria;
(f) said reaction being a toxic singlet oxygen reaction.

31. The process of claim 30 wherein said bacteria is E. coli.

32. A therapeutic process comprising:
(a) inserting an otoscope into an infected ear canal;
(b) said otoscope having an optical egress in communication with said ear canal;
(c) causing a reaction in bacteria in said infected ear canal by transmission of laser radiation from said optical egress to said bacteria;
(d) said laser radiation consisting essentially of one or both of a first radiation and a second radiation, said first radiation being primarily in a first wavelength range of 865 nm to 875 nm, and said second radiation being primarily in a second wavelength range of 925 nm to 935 nm;
(e) said first radiation and/or said second radiation activating a chromophore in said bacteria and cooperating with said chromophore to destroy said bacteria;
(f) said reaction being a toxic singlet oxygen reaction.

33. A therapeutic process comprising:
(a) subjecting a diseased anatomical local to laser radiation;
(b) causing a reaction in bacteria in said diseased locale by transmission of laser radiation to said bacteria;
(c) said laser radiation consisting essentially of one or both of a first radiation and a second radiation, said first radiation being primarily in a first wavelength range of 865 nm to 875 nm, and said second radiation being primarily in a second wavelength range of 925 nm to 935 nm;
(d) said first radiation and/or said second radiation activating a chromophore in said bacteria and cooperating with said chromophore to destroy said bacteria;
(e) said reaction being a toxic singlet oxygen reaction.
